Europäisches Patentamt

European Patent Office

Office européen des brevets

(11) Publication number: **0 278 656 B1**

# EUROPEAN PATENT SPECIFICATION

(45) Date of publication of patent specification: **03.06.92**   (51) Int. Cl.⁵: **C07D 501/46**

(21) Application number: **88300847.6**

(22) Date of filing: **02.02.88**

(54) Process for preparing ceftazidime pentahydrate.

(30) Priority: **02.02.87 US 10252**

(43) Date of publication of application:
**17.08.88 Bulletin 88/33**

(45) Publication of the grant of the patent:
**03.06.92 Bulletin 92/23**

(84) Designated Contracting States:
**AT BE CH DE ES FR GB GR IT LI NL SE**

(56) References cited:
**EP-A- 0 166 580**
**FR-A- 2 426 695**
**FR-A- 2 466 467**

(73) Proprietor: **ELI LILLY AND COMPANY**
**Lilly Corporate Center**
**Indianapolis Indiana 46285(US)**

(72) Inventor: **Wirth, David Dale**
**1219 Lockwood Drive**
**Lafayette, IN 47905(US)**

(74) Representative: **Hudson, Christopher Mark et al**
**Erl Wood Manor**
**Windlesham Surrey GU20 6PH(GB)**

## Description

This invention relates to an improvement in the process for preparing the cephalosporin antibiotic known as ceftazidime. In particular, it relates to an improvement in the process for preparing the pentahydrate form of ceftazidime from the dihydrobromide form of ceftazidime.

The commercially successful, semi-synthetic cephalosporin antibiotic ceftazidime is also chemically named 1-[[7-[[(2-amino-4-thiazol-yl)[1-carboxy-1-methylethoxy)imino]acetyl]amino]-2-carboxy-8-oxo-5-thia-1-azabicyclo-[4.2.0]oct-2-ene-3-yl]methyl]pyridinium hydroxide inner salt or (6R,7R)-7-[(Z)-2-(2-aminothiazol-4-yl)-2-(2-carboxyprop-2-oxyimino)acetamido]-3-(1-pyridiniummethyl)ceph-3-em-4-carboxylate (see, for example, U.S. Patent No. 4,258,041).

An especially useful pharmaceutical form of ceftazidime is its pentahydrate form. Ceftazidime pentahydrate is described in U.S. Patent No. 4,329,453.

The preparation of semi-synthetic cephalosporin antibiotics such as ceftazidime in advantageous forms such as the pentahydrate generally involves several steps. Because product losses usually occur at each step, multistep processes are expensive. Improvements in such processes which increase product yield are, therefore, of great value.

This invention provides an improvement in the yield of ceftazidime pentahydrate when obtaining it from ceftazidime dihydrobromide. The improvement comprises using a secondary-amine-type ion-exchange resin to remove undesirable by-products such as bromide ions from the aqueous solution of ceftazidime after dissolution of the dihydrobromide, thereby allowing ceftazidime pentahydrate to be isolated in much higher yield.

Thus, this invention provides a process for preparing ceftazidime pentahydrate from ceftazidime dihydrobromide, characterized in that a secondary-amine-type ion-exchange resin in a water-immiscible organic solvent is added to an aqueous solution of ceftazidime dihydrobromide, the resulting mixture being stirred while the pH is maintained at or below about 6.0 until the by-products are absorbed on the resin, the ceftazidime being isolated in its pentahydrate form from the aqueous phase.

The advantage of this improved process is that it provides much higher yields of ceftazidime pentahydrate than previously could be obtained from ceftazidime dihydrobromide. The process provides an improvement in yield whether or not a polystyrene resin treatment is also used. Yield improvements in the range of up to 25% have been observed.

Secondary-amine-type ion-exchange resins such as Amberlite LA-2 have been used in the purification of cephalosporins, including ceftazidime (see, for example, U.S. Patent No. 4,258,041, Examples 8 and 12) and ceftazidime bishydrochloride (see U.S. Patent No. 4,329,453, Example 1). In such instances, however, the resins either were used in a different way, i.e., bromide ion removal was not involved, or showed no advantage over similar isolation methods. For example, the yields obtained using the procedures of Examples 1, 2, 3 and 4 in U.S. 4,329,453 were about the same.

Secondary-amine-type ion-exchange resins which are suitable for the process of this invention are liquid and have a high molecular weight. Such resins are oil soluble, but water insoluble. Preferably, the resin will have a molecular weight in the range of 350-400 and a capacity of approximately 2.5-2.8 meq/g (2.1-2.3 meq/mL). Examples of resins which can be used are Amberlites LA-1 and LA-2 (Rohm & Haas, Philadelphia, PA 19105).

The ceftazidime dihydrobromide used in the process of this invention may be either the dihydrobromide itself or the dihydrobromide in a hydrated form such as the dihydrobromide monohydrate or dihydrate.

In carrying out the process of this invention, a solution of the ion-exchange resin in a suitable solvent is commingled with an aqueous solution of the ceftazidime dihydrobromide, preferably by adding the resin to the solution of the antibiotic. The solvent in which the ion-exchange resin is presented can be selected from a number of water-immiscible organic solvents. Examples of suitable solvents are dichloromethane, chloroform, ethyl acetate and methyl ethyl ketone. A preferred solvent is dichloromethane.

When the ion-exchange resin is mixed with the aqueous ceftazidime dihydrobromide solution, the resin will raise the pH of the solution. Care should be taken to maintain the pH of the resulting solution at or below about 6.0 by adding appropriate amounts of acid or base. Phosphoric acid is a particularly suitable acid for lowering the pH.

After the resin is added, the resulting mixture should be stirred to permit adsorption of by-products and bromide ions by the resin. The length of time the mixture is stirred is not critical and will vary, depending on the volume of the solution and the amount of resin used. In general, the mixture should be stirred for at least from about 1 to about 20 minutes.

For best results from this process, the aqueous ceftazidime solutions should be chilled. A preferred temperature range is from about 5 to about 15°C. A range of from about 8 to about 12°C is especially preferred.

Following the use of the liquid ion exchange resin, the ceftazidime can be recovered in its pentahydrate form from the aqueous layer by standard procedures, such as those described in U.S. Patent No. 4,329,453.

The following examples further illustrate the process of this invention.

## Example 1

Preparation of Ceftazidime Pentahydrate from Ceftazidime Dihydrobromide

### a. Prior Ceftazidime Pentahydrate Process

Ceftazidime dihydrobromide (potency 70.6%, 21.26 g) was added to deionized water (30 mL) which had been prechilled to 10°C. Sodium hydroxide (4 M) was added dropwise to raise the pH to 2.1 at 10°C. The solution was stirred 20 min at 10°C and filtered across a pad of filter aid. The pad was rinsed with water (10 mL), and the aqueous solutions were combined.

The pH of the combined solution was raised to 6.0 with 4 M sodium hydroxide, and the resulting solution was eluted across Dianion HP-20 resin (45 mL) which had been prechilled to ca. 10°C. The total volume collected from the resin, including the rinse of dionized water, was 120 mL.

Crystallization was induced by adjusting the pH to 4.0 with 4 M phosphoric acid and adding seed crystals. After $1\frac{1}{2}$ hr, more phosphoric acid was added over a 2-hr period to achieve a pH of 3.7. The pH was kept at 3.7 and the temperature was lowered to 0-5°C for 1 hr while the product crystallized. The product was collected by filtration, rinsed with cold water and cold acetone, and dried in vacuo at ambient temperature. This procedure gave 11.94 g of ceftazidime pentahydrate. The assay was 84.4%, and the corrected yield was 67.2%. A typical amount lost in the mother liquor by this procedure is 21%.

### b. Improved Ceftazidime Pentahydrate Process

Ceftazidime dihydrobromide (70.6% potency, 21.26 g) was added to deionized water (30 mL) which had been chilled to 10°C. Sodium hydroxide (4 M) was added dropwise to raise the pH to 2.0 at 10°C. The slurry was stirred at 10°C for 20 to 30 min to insure complete dissolution and then filtered across a small pad of filter aid. The pad was rinsed with deionized water (10 to 15 mL). The aqueous solutions were combined and chilled to 5°C.

Amberlite LA-2 (30 mL) was diluted with dichloromethane (120 mL) and washed sequentially with 0.4 M sodium hydroxide (150 mL) and a 3% aqueous solution of sodium chloride (150 mL). The resulting solution was added over a 5-min period to the ceftazidime solution, keeping the temperature at or below 12°C. The pH was prevented from rising above 6.0 by the addition of 85% phosphoric acid (0.5 mL).

After this mixture was stirred for 2 min at 10°C and pH 5.9, the layers were separated. The aqueous solution was washed with dichloromethane (140 mL) and then evaporated under vacuum to remove residual dichloromethane, giving a clear pale-yellow solution. This solution was then eluted across a column of Dianion HP-20 (40 mL) which had been prewashed with ice-cold deionized water (100 mL). The HP-20 column was rinsed with water (20 mL) which had been used to back-extract the combined dichloromethane solutions. The total column eluant collected was 120 mL.

The pH of the ceftazidime solution was lowered to 4.5 with 4 M phosphoric acid, and the solution was seeded. The solution was stirred for $2\frac{1}{2}$ hr at 0-5°C, during which the pH had risen to 4.7 and a large amount of crystals had formed. Phosphoric acid (4 M) was then added dropwise over a 1-hr period to achieve a pH of 3.7. More acid was added dropwise over the next $\frac{1}{2}$ hr to maintain this pH. After the pH was stabilized, the slurry was stirred slowly in an ice bath for 1 hr. The crystals were filtered and washed twice with cold deionized water (2 × 30 mL) and then with cold acetone (30 mL). The product was dried overnight at ambient temperature at a pressure of about 80 mm Hg. This procedure gave 13.57 g of ceftazidime pentahydrate. The assay was 85.4%, and the corrected yield was 77.3%. Another 11.1% yield was obtained from the combined mother liquors and washes.

## Example 2

Alternate Preparation of Ceftazidime Pentahydrate from Ceftazidime Dihydrobromide

The improved process of Example 1 was repeated except that Dianion HP-20 resin was not used. The aqueous ceftazidime solution obtained after the evaporation step was crystallized immediately by lowering the pH of the solution to 4.6 and proceeding as described in Example 1.

This procedure gave 15.92 g of ceftazidime pentahydrate. The assay was 84.4%, and the corrected yield was thus 89.6%. Another 7.7% yield was present in the mother liquor.

## Claims

1. A process for preparing ceftazidime pentahydrate from ceftazidime dihydrobromide, characterized in that a secondary-amine-type ion-exchange resin in a water-immiscible organic solvent is added to an aqueous solution of ceftazidime dihydrobromide, the resulting mixture being stirred while the pH is maintained at or below about 6.0 until the by-products are adsorbed on the resin, the ceftazidime being isolated in its pentahydrate form from the aqueous phase.

2. A process of claim 1 wherein the water-immiscible solvent is dichloromethane.

3. A process of claim 1 or 2 wherein the aqueous solution of ceftazidime dihydrobromide is chilled to a temperature of from about 5 to about 15°C.

**Revendications**

1. Procédé pour préparer le pentahydrate de la ceftazidime à partir de dibromhydrate de ceftazidime, **caractérisé en ce qu'**on ajoute une résine échangeuse d'tons du type amine secondaire dans un solvant organique non miscible à l'eau, à une solution aqueuse de dibromhydrate de ceftazidime, le mélange résultant étant agité tandis que l'on maintient le pH à ou en dessous d'environ 6,0 jusqu'à adsorption des sous-produits sur la résine, la ceftazidime étant isolée dans sa forme pentahydrate à partir de la phase aqueuse.

2. Procédé selon la revendication 1, dans lequel le solvant non miscible à l'eau est le dichlorométhane.

3. Procédé selon la revendication 1 ou 2, dans lequel on refroidit la solution aqueuse du dibromhydrate de ceftazidime à une température d'environ 5 à environ 15°C.

**Patentansprüche**

1. Verfahren zur Herstellung von Ceftazidimpentahydrat aus Ceftazidimdihydrobromid, dadurch gekennzeichnet, daß ein lonenaustauschharz vom Typ sekundäres Amin in einem mit Wasser nicht mischbaren organischen Lösungsmittel zu einer wäßrigen Lösung von Ceftazidimdihydrobromid zugegeben wird, die entstehende Mischung gerührt wird, während der pH auf oder unter etwa 6,0 gehalten wird, bis die Nebenprodukte an dem Harz adsorbiert sind, und das Ceftazidim in seiner Pentahydratform aus der wäßrigen Phase isoliert wird.

2. Verfahren nach Anspruch 1, worin das mit Wasser nicht mischbare Lösungsmittel Dichlormethan ist.

3. Verfahren nach Anspruch 1 oder 2, worin die wäßrige Lösung von Ceftazidimdihydrobromid auf eine Temperatur von etwa 5 bis etwa 15°C gekühlt wird.